# EUROPEAN PATENT SPECIFICATION

(11) **EP 0 287 313 B1**
(45) Date of publication and mention of the grant of the patent: **04.01.1995**
(21) Application number: 88303248.4
(22) Date of filing: 12.04.1988
(51) Int. Cl.: C07H 19/16, C07H 19/06, A61K 31/70

(54) **Acid stable dideoxynucleosides active against the cytopathic effects of human immunodeficiency virus**
Säurebeständige Dideoxynucleoside, wirksam gegen den HIV-Virus
Didéoxynucléosides, stables en milieu acide, actifs contre le virus d'immunodéficience humain

(30) Priority: 17.04.1987 US 39402
(43) Date of publication of application: 19.10.1988
(73) Proprietor: THE UNITED STATES OF AMERICA as represented by the Secretary United States Department of Commerce, Springfield, Virginia 22161 (US)
(72) Inventor: Marquez, Victor E., Bethesda Maryland 20892 (US); Driscoll, John S., Bethesda Maryland 20892 (US); Tseng, Christopher Kuo-Hou, Bethesda Maryland 20892 (US)
(74) Representative: Daley, Michael John

(56) References cited:
- CHEMICAL ABSTRACTS, vol. 95, 1981, page 726, abstract no. 204370s, Columbus,Ohio, US; & JP-A-81 86 200 (YAMASA SHOYU CO., LTD) 13-07-1981
- CHEMICAL ABSTRACTS, vol. 108, 1988, page 14, abstract no. 199v, Columbus, Ohio,US; V.E. MARQUEZ et al.: "2',3'-Dideoxy-2'-fluoro-ara-A. An acid-stable purinenucleoside active against human immunodeficiency virus (HIV)",& BIOCHEM. PHARMACOL. 1987, 36(17), 2719-22
- JOURNAL OF MEDICINAL CHEMISTRY, vol. 30, 1987, pages 2131-2137, AmericanChemical Society, Columbus, Ohio, US; P. HERDEWIJN et al.: "Synthesis and anti-HVI activity of various 2'- and 3'-substituted 2',3'-dideoxyadenosines: astructure-activity analysis"
- J. Med. Chem. 1990, 33, 978-983.

## Description

### FIELD OF THE INVENTION

The present invention relates to dideoxynucleosides which are stable in acid environments, such as found in the human stomach.

### BACKGROUND OF THE INVENTION

Acquired immune deficiency syndrome, or AIDS, is a fatal disease which has reached epidemic proportions among certain high risk groups. Several features of AIDS make therapy extremely difficult. The main target of the AIDS virus, now known as HIV, or human immunodeficiency virus, is the T4 lymphocyte, a white blood cell that marshals the immune defenses. This depletion of T4 cells in AIDS causes a severe depression of the immune response, so that a compound which is to be effective against AIDS must modify virus effect without much help from host immunity. Furthermore, the virus also affects cells in the central nervous system, where it is protected by the blood-brain barrier from compounds that might otherwise be effective against the virus. In infecting its host, the HIV binds to specific cell-surface receptor molecules. The virus penetrates the cell cytoplasm and sheds its protein coat, thereby baring its genetic material, a single strand of RNA. A viral enzyme, reverse transcriptase, accompanies the RNA. The virus, a retrovirus, reverse transcribes the RNA into DNA. Ultimately, some DNA copies of the HIV genome become integrated into the chromosomes of the host cell.

This integrated viral genome, known as a provirus, may remain latent until the host cell is stimulated, such as by another infection. The proviral DNA is then transcribed into mRNA, which directs the synthesis of viral proteins. The provirus also gives rise to other RNA copies that will serve as the genetic material of viral progeny. The proteins and the genomic RNA congregate at the cell membrane and assemble to form new HIV particles, which then break off from the cell. Two HIV genes, tat and trs/art, appear to control this burst of replication, which destroys the cell. These genes code for small proteins that boost the transcription of proviral DNA and the synthesis of viral proteins.

Several compounds have been shown to reduce the activity of reverse transcriptase in vitro. The reverse transcription is the step that is essential to viral replication and irrelevant to host cells. It has been found that HIV replication is considerably slower in the presence of compounds such as suramin, antimoniotungstate, phosphonoformate, and a class of nucleoside analogues known as dideoxynucleosides.

Nucleoside analogues are a class of synthetic compounds that resemble the naturally occurring nucleosides, which are chemical precursors of DNA and RNA. A nucleoside comprises a single-or double-ring base linked to a five-carbon sugar molecule. An analogue differs from the naturally-occurring nucleoside in large or small features of the base or the sugar. An enzyme that normally acts on a nucleoside in the course of viral replication can also bind to the nucleoside analogue. Because the nucleoside and the analogue differ, however, binding to the analogue can incapacitate the enzyme, thereby disrupting a molecular process crucial to viral replication.

Of the synthetic nucleoside analogues, 2',3'-dideoxyadenosine (ddA) has been found to have potent in vitro activity against the human immunodeficiency virus which causes AIDS. Because the activated form of dideoxynucleosides (5'-triphosphate) appears to inhibit the replication of the virus at the stage of reverse transcription of de novo infection of the virus, it is most likely that a drug of this type must be taken continuously if the therapeutic effect is to be maintained. Since daily treatment for a long period might ensue, oral drug administration is envisioned as the most practical route for a patient population numbering in the thousands.

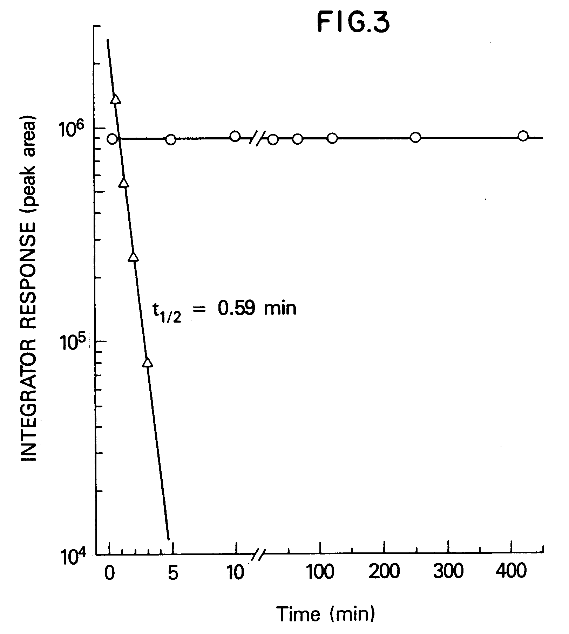

Drugs administered orally are exposed to a pH range of 1 to 2 in the human stomach environment for approximately one hour. This could result in drug stability problems with ddA, since this compound undergoes acid-catalyzed hydrolysis of the glycosidic bond at a rate 40,000 times faster than adenosine. It was found that ddA has a t_{1/2} of 35 seconds at pH 1.0 at 37°C (Figure 3). Cleavage of this compound not only reduces it efficacy, but potential problems of toxicity may occur due to formation of excessive quantities of the cleavage products.

### SUMMARY OF THE INVENTION

It is an object of the present invention to provide synthetic nucleosides which are useful in treating AIDS.

It is another object of the present invention to provide synthetic nucleosides which are stable in an acid environment.

It is a further object of the present invention to provide synthetic nucleosides which inhibit the infectivity of the human immunodeficiency virus.

It is yet a further object of the present invention to provide synthetic nucleosides which can be administered orally to treat acquired immune deficiency syndrome.

It is yet another object to overcome deficiencies in the prior art, such as indicated above; and still a further object to advance AIDS therapy.

The compounds of the present invention have potent activity in vitro against the HIV virus, the virus which causes AIDS. These compounds are also stable to the acidic conditions which exist in the human stomach, pH 1-2.

The compounds of the present invention have the following formulae: 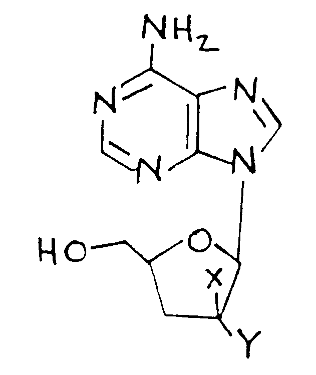
wherein
X is selected from the group consisting of hydrogen, F, CN, and NO₂;
Y is either H or F; with the proviso that X and Y cannot both be H.

The compound obtained when X and Y are both H is 2',3'-dideoxy-adenosine, referred to hereinafter as Compound I, a known compound.

The compound obtained when X=H and Y=F is 2',3'-dideoxy-2'-alpha-fluoroadenosine, hereinafter referred to as Compound II. The compound obtained when X=F and Y=H is 2',3'-dideoxy-2'-beta-fluoroadenosine, hereinafter referred to as Compound III.

Another embodiment of the invention is a compound represented by the formula: 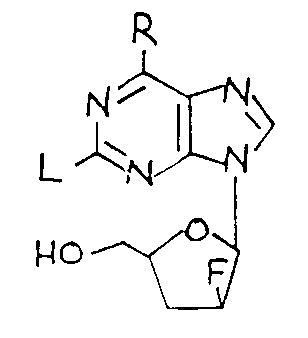
wherein
R is either OH or NH₂; and
L is NH₂.

Still another embodiment of the invention is a compound represented by the formula: 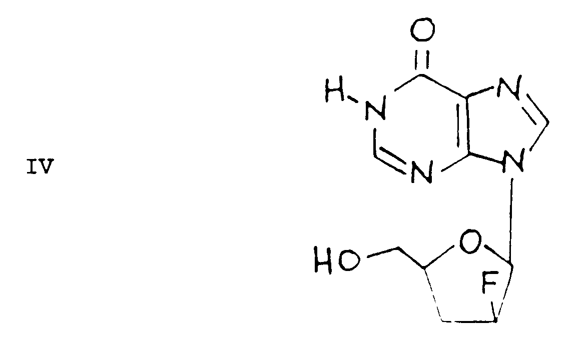
In yet another aspect, the present invention relates to compounds of the formula: 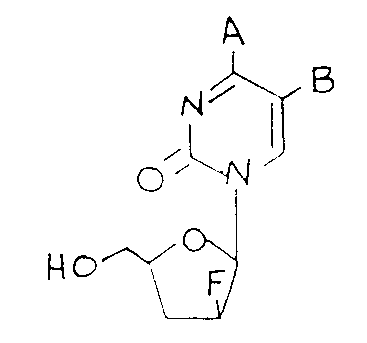
wherein
A is selected from OH and NH₂;
B is selected from the group consisting of H, CH₃, CF₃, CH=CHBr, and halogen.

### DESCRIPTION OF THE DRAWINGS

Figure 1 shows the activity and potency of ddA and the 2'-beta-fluoro derivative (Compound III) of ddA against HIV.

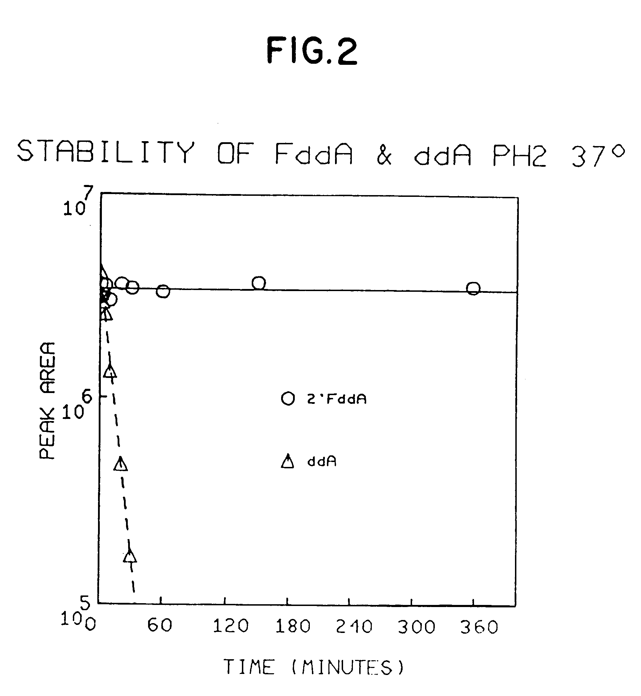

Figure 2 shows the stability of 2,3-dideoxyadenosine (Compound I) and 2',3'-dideoxy-2'-alpha-fluoroadenosine (Compound II) in an environment of pH 2 at 37°C.

Figure 3 shows the rate of decomposition for 2',3'-dideoxyadenosine (△) and 2',3'-dideoxy-2-betafluoroadenosine (○) at pH 1 and 37°C.

### DETAILED DESCRIPTION OF THE PREFERRED EMBODIMENTS OF INVENTION

In order to study acid-catalyzed decomposition of the subject compounds, a pH 1 buffer was prepared by dissolving 0.746 g KC1 and 26.8 g 1.0 N HC1 in sufficient distilled water to give 200 ml total volume. Ten ml of buffer, prewarmed to 37°C., was added to 102 micrograms of ddA (Compound I), and 93 micrograms of each of compounds II or III. The samples were shaken and maintained at 37°C. Aliquots were taken at timed intervals and neutralized immediately with 0.1 NaOH and chilled on ice. The amounts of ddA and the fluorinated analogs were determined by HPLC analysis using a 4.6 x 250 mm 5 microgram Ultrasphere ODS column protected by a guard column packed with 37-50 micron Vydac 201SC. Elution was with 12% acetonitrile in 0.01 M pH 6.8 phosphate buffer at 1.0 ml/minute. The integrator peak areas were plotted as a function of time, and the data was fitted to a first order decomposition curve by a computer program (MLAB).

To test the cytopathic effect of the compounds of the present invention, HIV cytopathic assay was performed using ATH8 cells as described by Mitsuya et al., Proc. Nat. Acad. Sci. USA, 83, 1911 (1986). In brief, 2 x 10⁵ ATH8 cells were preexposed to polybrene, exposed to HTLV-III_{B} virus (2000 virus particles/cell) for 45 minutes after treatment with polybrene, resuspended in 1 ml of culture medium containing Interleukin 2 in the presence or absence of various concentrations of compounds, and incubated in culture tubes at 37°C. in 5% CO₂/95% air humidified atmosphere. Control cells were treated similarly but were not exposed to the virus. At various times on days 5 to 7 of culture, the total viable cells were counted in a hemocytometer by the trypan blue dye exclusion method. In the HIV cytopathic effect assay using ATH8 cells, 0.5 to 5 virus particles per cell represent the minimum cytopathic dose of the virus.

The 2'-alpha- and 2'-beta-fluoroisomers of 2',3'-dideoxyadenosine, compounds II and III, were synthesized as described below.

The alpha-isomer was obtained in four steps from 3'-deoxy-ara-A. The 5'-hydroxyl group was protected with dimethoxytrityl chloride, and the 2'-hydroxyl group was activated by formation of the corresponding triflate derivative. The configuration of the 2'-position was inverted by an SN₂ displacement using tetra-n-butylammonium fluoride. The dimethoxytrityl protective group was removed with dichloroacetic acid. The nucleophilic displacement of triflate by fluoride ion gave compound II as a lyophilized white powder, R_{f} 0.25 (SiO₂, CHC1₃ :CH₃OH ::9:1); m/z calc. C₁₀H₁₃N₅O₂F (MH+)254.1052, found 254.1059 ± 0.0017. This was accompanied by a minor, olefin-forming reaction caused by elimination rather than displacement of the triflate group. A similar type of elimination reaction became exclusive when the same synthetic approach was applied to the preparation of the beta-isomer from 3'-deoxyadenosine (corcycepin).

The failure of the triflate displacement reaction required the development of an alternative approach for formation of the beta-isomer. This required the synthesis of the previously reported compound 6-amino-9-(beta-D-2'-deoxy-2'-fluoroarabinofuranosyl)-9-H-purine (2'-F-ara-A, compound VI), as a starting material. This intermediate, originally synthesized by Fox et al., J. Org. Chem., 34, 2632 (1969) was prepared using the improved general procedure of Montgomery et al., J. Med. Chem., 29, 2389 (1986). This improved procedure involved condensing 6-chloropurine with 3-O-acetyl-5-O-benzoyl-1-2-deoxy-2-fluoro-D-arabinofuranosyl bromide. The required functionalized halogenosugar was prepared in essentially the same manner as reported by Fox et al., J. Carbohyd. Res., 42, 233 (1975). As expected, four isomers were obtained from the condensation reaction. After separation and characterization of the correct 6-chloro isomer, the required starting material, 2'-F-ara-A, was obtained by ammonolysis with concentrated methanolic ammonia which simultaneously removed the protective groups. All of the chemical, optical, and spectral properties of the compound matched those reported for 2'-F-ara-A. Selective protection of the 5'-hydroxyl function of this compound by reaction with t-butyldimethyl silyl chloride gave a product that permitted the two-step reduction of the 3'-hydroxyl group. Treatment with phenyl chlorothiocarbonate followed by reaction of the intermediate 3'-O-phenoxythiocarbonyl derivative with tri-n-butyl tin hydride, produced the desired 2',3'-dideoxy nucleoside with the 2'-fluorine in the beta, or "up", configuration. This required only the removal of the 5'-blocking group with tetra n-butyl ammonium fluoride to give compound III as a white lyophilized product, R_{f} 0.18 (SiO₂, CHC1₃ :CH₃OH: :9:1); m/z calc. C₁₀H₁₃N₅O₂F (MH⁺) 254.1052, found 254.1031 ± 0.0018.

It was found that 2',3'-dideoxy-alpha-fluoroadenosine, compound II, was stable to acid-catalyzed decomposition; the addition of a fluorine atom in this "down" configuration gave the compound with a protective effect against HIV of 25% that seen with ddA.

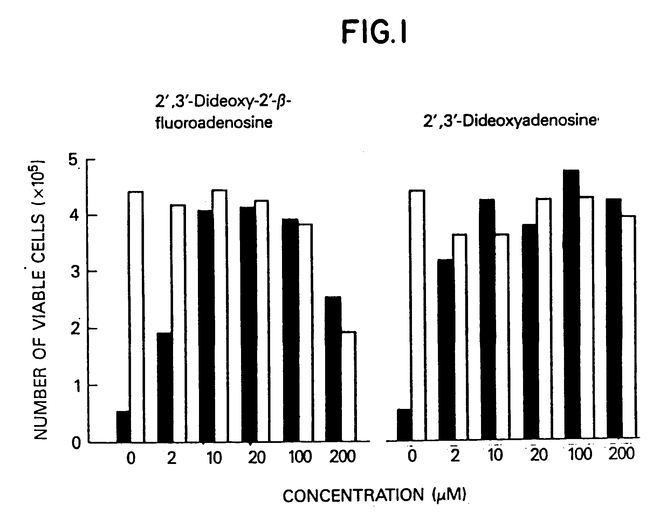

A change in the fluorine stereochemistry at the 2'-position, however, produced dramatically better results. Compound III, 2',3'-dideoxy-2'-beta-fluoroadenosine was approximately as active and potent as ddA in protecting ATH8 cells against the cytopathic effect of HIV, as shown in Figure 1, under conditions of substantial viral excess. Furthermore, the antiviral effect of compound III was as durable as that of the parent compound, compound I. In addition, compound III was completely unchanged after a 24 hour exposure to acidic conditions at pH 1, as shown in Figure 3. Based on the mechanism proposed for the acid decomposition of this compound, the increased stability of this compound as well as that of the alpha-isomer may be a consequence of the inductive destabilization by fluorine of the intermediate oxonium ion which would result from hydrolysis of the glycosidic bond. While fluorine is an ideal group to introduce because of its strong electronegative effect and size similarity to hydrogen, other electronegative groups in the 2'-position such as cyano, azido, nitro etc., should have acid stabilizing properties also.

2',3'-dideoxy-2'-beta-fluoroinosine, compound IV, was prepared enzymatically from compound III, 2',3'-dideoxy-2'-beta-fluoroadenosine, by treatment with adenosine deaminase (adenosine aminohydrolase, EC 3.5.4.4). One milligram of 2',3'-dideoxy-2'-beta-fluoroadenosine was dissolved in one ml water at room temperature, and 0.25 microliter of commercial adenosine deaminase enzyme solution, one unit, was added. The reaction, which was monitored by HPLC (260 nm, UV detection), was complete in one hour.

Compound IV, 2',3'-dideoxy-2'-beta-fluoroinosine, has a HPLC retention time of 6.64 minutes using a 4.6 x 250 mm 5 microliter Beckman/Altex Ultrasphere ODS analytical column preceded by a Waters guard column (Vydak). The mobile phase used was 8.5% acetonitrile/0.01 M phosphate buffer, at pH 6.8. Compound IV was obtained by ultrafiltration to remove the protein, followed by lyophilization.

It has been found that adenosine deaminase in the body causes the metabolism of 2',3'-dideoxy-2'-beta-fluoroadenosine to compound IV, 2',3'-dideoxy-2'-beta-fluoroinosine. Compound IV is also useful in inhibiting the infectivity of the HIV virus.

Because the compounds of the present invention are stable in an acid environment such as is found in the human stomach, they can readily be formulated without the need for pH buffers into dosages suitable for oral administration, using pharmaceutically acceptable carriers, which carriers are well known in the art. Such carriers may enable the compounds to be formulated as tablets, pills, capsules, liquids, gels, and the like, for oral ingestion by a patient to be treated for AIDS.

The precise dosage amounts to be administered will be determined by routine experimentation. In general, however, the dosage amounts will be comparable or less than those already known from the experimental use of dideoxy adenosine.

## Claims

1. A compound characterized in that it conforms to the formula: 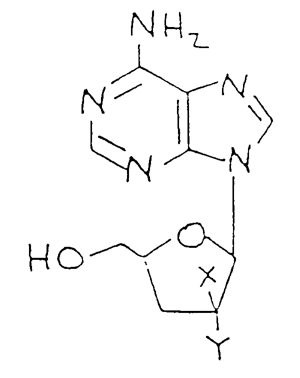 wherein
X is selected from
hydrogen, F, CN, and NO₂;
Y is either H or F; with the proviso that X and Y cannot both be H.

2. A compound according to claim 1 which is 2',3'-dideoxy-2'-beta-fluoroadenosine (compound III)

3. A compound according to claim 1 which is 2',3'-dideoxy-2'-alpha-fluoroadenosine (compound II)

4. A composition for inhibiting the infectivity of the human immunodeficiency virus characterized in that it comprises a compound according to any one of claims 1 to 3 and a pharmaceutically acceptable carrier.

5. A process for preparing 2',3'-dideoxy-2'-beta-fluoroinosine (compound IV) characterised in that it comprises treating 2',3'-dideoxy-2'-beta-fluoroadenosine with adenosine deaminase.

6. A compound characterized in that it conforms to the formula 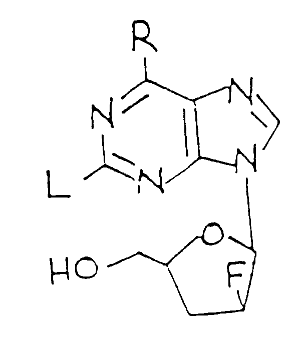 wherein
R is selected from OH and NH₂
L is NH₂.

7. A compound characterized in that it is 2',3'-dideoxy-2'-beta-fluoroinosine (compound IV).

8. A composition for inhibiting the infectivity of the human immunodeficiency virus characterized in that it comprises the compound of claim 7 and a pharmaceutically acceptable carrier therefor.

9. The compound characterized in that it conforms to the formula: 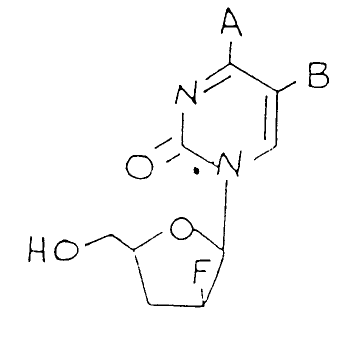 wherein
A is selected from OH and NH₂;
B is selected from H, CH₃, CF₃, CH=CHBr, and halogen.

10. A composition for inhibiting the infectivity of the human immunodeficiency virus characterised in that it comprises a compound according to any of claims 9 and a pharmaceutically acceptable carrier therefor.

## Patentansprüche

1. Verbindung, dadurch gekennzeichnet, daß sie der Formel entspricht: 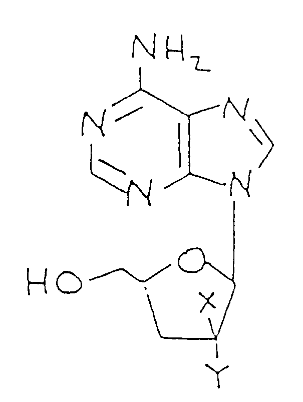 wobei X aus Wasserstoff, F, CN, und NO₂ ausgewählt ist, Y entweder H oder F ist, mit der Maßgabe, daß X und Y nicht beide H sein können.

2. Verbindung nach Anspruch 1, die 2',3'-Didesoxy-2'-Beta-Fluoradenosin (Verbindung III) ist.

3. Verbindung nach Anspruch 1, die 2',3'-Didesoxy-2'-Alpha-Fluoradenosin (Verbindung II) ist.

4. Zusammensetzung zur Unterdrückung der Infektivität des Human-Immundefektvirus, dadurch gekennzeichnet, daß sie eine Verbindung nach einem der Ansprüche 1 bis 3 einen pharmazeutisch annehmbaren Träger enthält.

5. Verfahren zur Herstellung von 2',3'-Didesoxy-2'-Beta-Fluorinosin (Verbindung IV), dadurch gekennzeichnet, daß es die Behandlung von 2',3'-Didesoxy-2'-Beta-Fluoradenosin mit Adenosin-Desaminase umfaßt.

6. Verbindung, dadurch gekennzeichnet, daß sie der Formel entspricht: 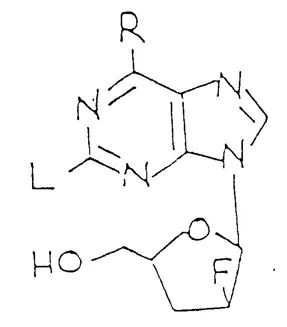 wobei R aus OH und NH₂ ausgewählt ist und L als NH₂ vorliegt.

7. Verbindung, dadurch gekennzeichnet, daß sie 2',3'-Didesoxy-2'-Beta-Fluorinosin (Verbindung IV) ist.

8. Zusammensetzung zur Unterdrückung der Infektivität des Human-Immundefektvirus, dadurch gekennzeichnet, daß sie die Verbindung nach dem Anspruch 7 und einen pharmazeutisch annehmbaren Träger hierfür enthält.

9. Verbindung, dadurch gekennzeichnet, daß sie der Formel entspricht: 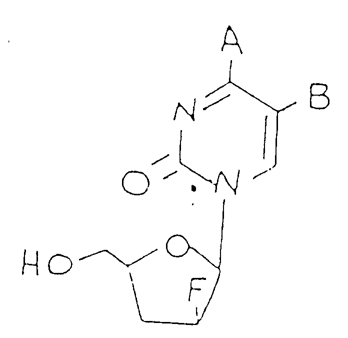 wobei A aus OH und NH₂ ausgewählt ist, und B aus H, CH₃, CF₃, CH=CHBr, und Halogen ausgewählt ist.

10. Zusammensetzung zur Unterdrückung der Infektivität des Human-Immundefektvirus, dadurch gekennzeichnet, daß sie eine Verbindung nach Anspruch 9 und einen pharmazeutisch annehmbaren Träger hierfür enthält.

## Revendications

1. Composé caractérisé en ce qu'il répond à la formule : 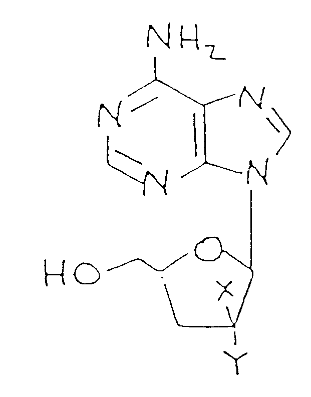 dans laquelle
X est choisi parmi l'hydrogène, F, CN, et NO₂;
Y est soit H, soit F, à la condition que X et Y ne soient pas tous les deux H.

2. Composé selon la revendication 1 consistant en la didésoxy-2',3'-béta-fluoro-2'-adénosine (composé III).

3. Composé selon la revendication 1 consistant en la didésoxy-2',3'-alpha-fluoro-2'-adénosine (composé II).

4. Composition pour inhiber le pouvoir infectieux du virus de l'immunodéficience humaine, caractérisée en ce qu'elle comprend un support pharmaceutiquement acceptable.

5. Procédé pour préparer la didésoxy-2',3'-béta-fluoro-2'-inosine (composé IV), caractérisé en ce qu'il comprend l'étape de traitement de la didésoxy-2',3'-béta-fluoro-2'-adénosine avec l'adénosine désaminase.

6. Composé caractérisé en ce qu'il répond à la formule : 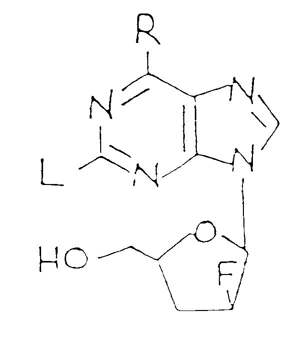 dans laquelle
R est choisi parmi OH et NH₂,
L est NH₂.

7. Composé caractérisé en ce qu'il consiste en la didésoxy-2',3-béta-fluoro-2'-inosine (composé IV).

8. Composition pour inhiber le pouvoir infectieux du virus de l'immunodéficience humaine caractérisée en ce qu'elle comprend le composé de la revendication 7 et un support pharmaceutiquement acceptable, pour ledit composé.

9. Composé caractérisé en ce qu'il répond à la formule : 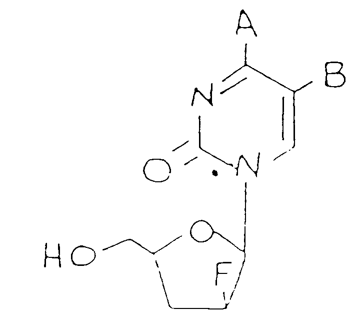 dans laquelle
A est choisi parmi OH et NH₂,
B est choisi parmi H, CH₃, CF₃, CH=CHBr, et les halogènes.

10. Composition pour inhiber le pouvoir infectieux du virus de l'immunodéficience humaine, caractérisée en ce qu'elle comprend un composé selon la revendication 9 et un support pharmaceutiquement acceptable pour ledit composé.
